(19)

Europäisches
Patentamt
European
Patent Office
Office européen
des brevets

(11) **EP 1 925 311 A1**

(12) **EUROPEAN PATENT APPLICATION**

(43) Date of publication:
**28.05.2008 Bulletin 2008/22**

(51) Int Cl.:
*A61K 36/185* (2006.01)　　*A61K 36/53* (2006.01)
*A61P 3/10* (2006.01)

(21) Application number: **06450165.3**

(22) Date of filing: **15.11.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(71) Applicant: **Melbrosin International Produktions
und
Vertriebs GmbH & Co KG
1090 Wien (AT)**

(72) Inventors:
• **Jungbauer, Alois
  1210 Vienna (AT)**
• **Müller, Monika
  3250 Wieselburg (AT)**
• **Zöchling, Alfred
  2523 Tattendorf (AT)**

(74) Representative: **Sonn & Partner Patentanwälte
Riemergasse 14
1010 Wien (AT)**

(54) **Use of a plant extract or plant juice**

(57)　　Use of a plant extract or plant juice for the production of a pharmaceutical or nutritional preparation for the treatment of metabolic syndrome, wherein the plant extract or juice is from thyme, oregano, glove, nutmeg, red clover, bay leaves or red onion, as well as chemical compounds usually obtainable from plants for this use.

EP 1 925 311 A1

**Description**

The present invention relates to the field of plants and plant extracts with medical or pharmaceutical use.

Metabolic syndrome is a combination of medical disorders that affect a large number of people in a clustered fashion. The end result of which is to increase one's risk for cardiovascular disease and diabetes. In most cases metabolic syndrome culminates in type 2 diabetes. The symptoms of metabolic syndrome are related to lipid and carbohydrate metabolism and include obesity, elevated triglycerides, low levels of high density lipoproteins, increased blood pressure or hypertension and increased glucose levels, but also symptoms of inflammation (Grundy, 2006). Generally, the individual symptoms associated with the metabolic syndrome are treated separately (e.g. diuretics and ACE inhibitors for hypertension, statins to decrease cholesterol levels or glitazones to treat diabetes). The problem is compounded when multiple drugs are necessary to control multiple risk factors. For example, once type 2 diabetes develops in patients with the metabolic syndrome, patients often require 10 or more drugs for treatment.

Novel potentially efficacious class of drugs for the metabolic syndrome as a whole, as well as for patients with type 2 diabetes, are the thiazolidinediones (TZDs). These drugs act by agonizing the nuclear receptor PPAR-gamma, which is predominantly expressed in adipose tissue, but also occurs in other tissues (Grundy, 2006; WO 2005/027661; CA 2 526 589). TZDs reduce the secretion of unesterified fatty acids and adipokines such as tumour-necrosis factor-alpha (TNF-alpha), other inflammatory cytokines, resistin and plasminogen-activator inhibitor 1 (PAI1); they also enhance adipose-tissue release of adiponectin. The net result of these changes, apparently, is to reduce insulin resistance in muscle and liver and to mitigate prothrombotic and pro-inflammatory states. These findings therefore suggest that TZDs are hitting at the heart of the metabolic syndrome by improving insulin resistance in adipose tissue. Still, in type 2 diabetes, they have only a modest effect on plasma lipoproteins and blood pressure. So, although they improve the metabolic syndrome, they by no means cure it once type 2 diabetes develops.

The PPAR-gamma is a class II nuclear receptor that forms a heterodimer with the retinoid X-receptor (RXR) and binds to specific regions on the DNA of target genes. Expression of target genes is increased or decreased, depending on the gene. PPARγ is composed of 6 structural regions in 4 functional domains. The A/B region forms the ligand-independent transactivation domain which can be covalently modified by phosphorylation. By the C region, the DNA-binding domain of the receptor can be targeted to the PPARγ response element (PPRE), a specific sequence of nucleotides within the regulatory region of responsive genes. The E/F region contains the ligand binding domain and the co-activator/co-repressor-binding surface. Binding of agonists leads to conformational changes of the receptor and to its activation. The activated receptor heterodimerizes with RXR and this heterodimer binds to PPRE through the DNA-binding domain (Guo, 2006).

Natural ligands of the PPAR-gamma receptor are fatty acids such as lauric acid, petroselenic acid, linolenic acid, linoleic acid and arachidonic acid, fatty acid metabolites like 15-deoxy-delta 12,14-prostaglandin J2. The synthetic ligands comprise the group of thiazolidinedions (Troglitazone, Rosiglitazone, Pioglitazone), the non-thiazolidinediones (e.g. GW1929, GW7845) and the non-steroidal anti-inflammatory drugs (e.g. flufenamic acid, fenoprofen).

PPAR-gamma downregulates TNF-alpha, leptin, IL-6, plasminogen activator inhibitor-1 (PAI-1), resistin, 11-beta-hydroxysteroid dehydrogenase type-1 (11-beta-HSD-1). Those proteins are responsible for insulin resistance. Thus activation of PPAR-gamma leads to reduced insulin resistance because they regulate the glucose-transporter protein GLUT-4 in the cell membrane.

Insulin resistance is a key factor in development of the metabolic syndrome. This syndrome is the coexistence of hyper-glycaemia, hypertension, dyslipidemia and obesity. Therefore cardiovascular diseases such as coronary heart diseases and stroke are more prevalent among patients with metabolic syndrome (Gurnell et al., 2003).

The WO 2005/053724 mentions non-aqueous extracts of Astragalus membranaceus which influence PPAR-gamma. Compounds found in these extracts are for example calycosin, formononetin, genistein, afromorsin, biochanin A, coumestrol, odoratin and daidzein. PPAR-gamma mediated diseases which can be treated with the extracts are dyslipidaemia, atherosclerosis, coronary heart disease, obesity and colon cancer.

The WO 2005/027661 describes catechines which is obtainable from green tea as activating ligand of PPAR-gamma. The ligands mentioned therein, in particular glitazone, rosiglitazone, ciglitazone and pioglitazone, fall under the group of TZDs. Further TZDs, for example troglitazone are mentioned in the US 2006/0030597 A1.

The CA 2 526 589 A1 describes ligands of PPAR-gamma, in particular glabrene, glabridine, glabrol and their derivatives, and glitazones. These compounds are mentioned in connection with the multiple risk factor syndrome, another name of the metabolic syndrome, which is related to insuline resistence and can be treated with PPAR-gamma ligands. Also described is a licorice extract for the treatment of metabolic syndrome.

The EP 1 350 516 B1 claims a hydrophobic licorice extract, and extracts from turmeric, clove and cinnamon for the use of treating metabolic syndrome as well as associated diseases like visceral obesity and diabetes mellitus. The activity of the extracts is measured in reference to troglitazone and pioglitazone.

The JP 2005/097216 mentions dehydrodieugenol A and B, magnolol, oleanic acid and betulic acid as PPAR-gamma ligands which are useful for preventing or ameliorating metabolic syndrome.

It is the goal of the present invention to provide a pharmaceutical preparation with particular exceptional potential to threat or prevent metabolic syndrome and diseases associated with metabolic syndrome.

Therefore the present invention provides the use of a plant extract or plant juice for the production of a pharmaceutical or nutritional preparation for the treatment or prevention of metabolic syndrome, wherein the plant extract or juice is from thyme, oregano, glove, nutmeg, red clover, bay leaves, red onion or grapes. The extracts activate the receptor PPARγ and thus induce a reduction of blood glucose. For example the extracts can be made with DMSO (dimethylsulfoxide) as extracting agent. The final extract or juice may have other solvents, preferably nutritional or pharmaceutically suitable solvents. It was now surprisingly found that preparations of extracts or juices of these plants can be used to treat metabolic syndrome. The term juice includes fermented juices or beverages such as wines, especially red wine made from grape vines, e.g. Vitis sp..

Rare mutations in the human PPAR-gamma are associated with lower transactivational capacity or complete loss of function lead to development of a certain type of familial lipodystrophy in the bearers, associated with all signs of metabolic syndrome. The PPAR-gamma agonist rosiglitazone has been shown to improve insulin resistance in such cases. Also genetically modified animals with loss of function of the PPAR-gamma exhibit features of the metabolic syndrome.

In contrast, impaired transactivation, which has been described in the frequent genetic polymorphism Ala12Pro of the PPAR-gamma gene, leads to higher insulin sensitivity (but lower postprandial hypertriglyceridemia), and a haplotype for which higher transcriptional activity is postulated has an increased risk for metabolic syndrome. Therefore, a non-linear activity-effect curve has been proposed for PPAR-gamma activity, with small increases in activity having opposite effects than stronger activation by ligands.

A lot of plants and plant extracts have hypoglycemic activity. Most prominent representatives of these plants/ plant extracts are: Cinnamon, cinnamon powder/cinnamon bark powder (*Cinnamomum verum),* bitter melon *(Momordica charantia),* cumin *(Cuminum cyminum),* tumeric *(Curcuma longa)* and fenugreek (Tri*gonella foenum-graecum*). These extracts are currently used or marketed for treatment of diabetes II. Other plant extracts have been mentioned to have hypoglycemic activities. These are: glove (Syzygium aromaticum), oregano (Origanum sp.), thyme (Thymus vulgaris), nutmeg (Myristica fragrans), alfalfa *(Medicago* sativa), red clover *(Trifolium pratense),* bay leaves *(Laurus no*bilis), white cabbage *(Brassica oleracea* var. *capitata* f. *alba),* curly hale *(Brassica oleracea* convar. *acephala* var. *sabellica)* and black pepper *(Piper nigrum).* In all above mentioned cases the activation of Peroxisome proliferator-activated receptor gamma was unknown.

Also disclosed herein is the method of treating a patient with metabolic syndrome or preventing metabolic syndrome with a preparation described herein. "Preventing" or "prevention" herein does not require absolute success in the sense of an absolute prevention but indicates a reduced risk of developing metabolic syndrome.

Preferably the plant extract or juice is from oregano. Oregano has been attributed anti-hyperglycemic activity (McCue, 2004; Yaniv, 1987; Lemhadri, 2004; McCue, 2004). It has been hypothesised that oregano extracts and rosmaric acid as a compound present in oregano inhibit pancreatic amylase and thus exhibit anti-hyperglycemic activity. Inhibition of starch break down to glucose contributes to the management of hyperglycemia and diabetes complication in the long term. It has been also mentioned that oregano inhibits aldose reductase, the first enzyme of the polyol pathway implicated in the secondary complications of diabetes (Koukoulitsa, 2006). However the indications were never strong enough to promote an activity against metabolic syndrome. The compounds in oregano have been thoroughly examined (see table 3 below). However it was found that the individual activity of the constituents in isolated form can only explain about 10% of the PPAR-gamma activity of oregano plant extracts. This takes the extreme divergence of oregano batches into account (see table 4). The PPAR-gamma activity of oregano strongly varies depending on growth factors like soil composition, light intake, and cutting time - these factors seem to be more important than the choice of oregano species and strain (see table 2).

Preferably the plant extract or juice has an $EC_{50}$-value of the PPAR-gamma binding of less than 50 $\mu$g/ml, preferably less than 35 $\mu$g/ml, more preferred less than 20 $\mu$g/ml, even more preferred less than 10 $\mu$g/ml, especially preferred less than 5 $\mu$g/ml, most preferred less than 1 $\mu$g/ml based on the dry-weight of the plant. These values can be estimated by the assay described in the example section and influenced as mentioned above by the selection of an appropriate batch in the case of strong activity variance as in oregano.

In another aspect of the present invention the use of isolated compounds selected from Quercetin, 2-Hydroxychalcone, Luteolin, Cinnamaldehyde, Diosmetin, 2'-Hydroxychalcone, Phloretin, Isoquercetin, Myricetin, Equol, Eriodictyol, ODMA (O-Desmethyl-angolenain), Resveratrol, Catechin, Apigenin, Vitexin, Gallic acid, Galangin, Naringin, Eugenol, Eriodictyol, Apigenin, Taxifolin, Salvianolic acid B, Chrysoeriol, Kaempferol, Thymol, Carvacrol, Safrol, Ethylcinnamate, Limonene or mixtures thereof, preferably selected from Quercetin, Luteolin, Diosmetin, Isoquercetin, Eridictoyl, Naringenin, Eriodictyol, Apigenin, Vitexin, Taxifolin, Salvianolic acid B, Chrysoeriol, Kaempferol, Thymol, Carvacrol or mixtures thereof, is provided for the production of a pharmaceutical or nutritional preparation for the treatment or prevention of metabolic syndrome. Especially preferred are those compounds found in oregano given in table 3 below or those from table 1. These compounds have been tested and could now for the first time be attributed with adequate PPAR-gamma binding activity which enables a use against metabolic syndrome. These compounds can be provided as a pharmaceutical or nutritional preparation for oral intake, for example as juice or as tablet. Additionally the plant extracts or juices can be selected to comprise one or more of these substances in a further embodiment.

In preferred embodiments the compounds of the preparation have an $EC_{50}$-value of the PPAR-gamma binding of less than 900 $\mu$M, preferably less than 500 $\mu$M, more preferred less than 150 $\mu$M, even more preferred less than 70 $\mu$M, especially preferred less than 55 $\mu$M, most preferred less than 15 $\mu$M. Adequate compounds can be selected alone or in combination, for example given the information of table 1 below.

All preparations (e.g. of the extract or of the compounds) described so far can be used to treat, ameliorate or prevent metabolic syndrome. Preferably the metabolic syndrome is associated with insuline resistence and/or a lack of activation of PPAR-gamma. Activity of PPAR-gamma can be determined in vitro with a sample with the Peroxisome Proliferator-Activated Receptor-gamma Competitor Assay Kit, Green (Invitrogen) as exemplified below. Stimulation of the PPAR-gamma with the extract, juice or active compound can circumvent malign effects of a lack of PPAR-gamma activation.

In further embodiments the metabolic syndrome is associated with diabetes, obesity, dyslipidaemia, hypolipidaemia, insuline resistence or arteriosclerosis. Cardiovascular diseases are a consequence of metabolic syndrome and can also be an associated indicator of metabolic syndrome. Symptoms of these diseases normally occur in the development of metabolic syndrome and can be treated with the inventive preparations. In particular the preparations are effective to treat the symptoms of diabetes type 2 since the PPAR-gamma activation can ameliorate glucose sensitivity. Therefore in a preferred embodiment the metabolic syndrome is associated with the clinical symptoms of diabetes type 2.

Additionally the present invention provides for a use of as described herein, wherein the extract, juice or compounds are used for the production of a pharmaceutical or nutritional preparation for the treatment of metabolic syndrome and/or at least one of selected from circulatory disorders and stenosis. It was surprisingly found that the preparations of the present invention can also be used for this combination treatment. Especially the oregano extracts increase the activity of eNOS (Endothelial Nitric Oxide Synthase). eNOS generates nitric oxide in blood vessels and is involved with regulating vascular function. eNOS is associated with plasma membranes surrounding cells and the membranes of Golgi bodies within cells. Nitric oxide is synthesized from arginine and oxygen. In the endothelium of blood vessels nitric oxide is the signal to the surrounding smooth muscle to relax, thus dilating the artery and increasing blood flow. This additional effects of the preparations allows the treatment of both metabolic syndrome and circulatory disorders (independently or in combination) with only one medicament. Therefore one aspect of the present invention is the use of preparation (of the extracts, juices or the active compounds) for increasing the activity of eNOS in a patient (human or animal) and for the treatment of eNOS associated diseases like circulatory disorders, cardiovascular disease and stenosis (also in combination with metabolic syndrome).

Preferably the extract, juice or compounds have an eNOS activity of 3 pMol/mg to 40 pMol/mg, preferably of 4 to 35 pMol/mg, even more preferred of 5 pMol/mg to 30 pMol/mg, most preferred of 6 pMol/mg to 25 pMol/mg. The eNos activity can be determined by the methods described below and can be selected by varying the constituents and their concentration in their preparation.

In a preferred embodiment, the pharmaceutical preparation according to the invention comprises a pharmaceutical carrier. A pharmaceutical carrier includes wetting, emulsifying, or pH buffering agents or vehicles with which the pharmaceutical preparation can be contained in or administered. Examples are oils, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Preferably, the agents of the pharmaceutical preparation are formulated as salt forms. Pharmaceutically acceptable salts include those formed with anions such as those derived from hydrochloric, phosphoric, acetic, oxalic, tartaric, butyric acids, etc., and those formed with cations such as those derived from sodium, potassium, ammonium, calcium, ferric hydroxides, isopropylamine, triethylamine, 2-ethylamino ethanol, histidine, procaine, etc.

Preferably the preparation is an oral preparation, in particular preferred in form of a juice or tablet. The extracts, juices or compounds can be dried and formulated into tablets and administered orally. For example, the preparations can be formulated in the form of tablets, capsules, cachets, gelcaps, solutions, suspensions, and the like. Tablets or capsules can be prepared by conventional means with pharmaceutically acceptable excipients such as binding agents (e.g., pregelatinized maize starch, polyvinylpyrrolidone, or hydroxypropyl methylcellulose); fillers (e.g., lactose, microcrystalline cellulose, or calcium hydrogen phosphate); lubricants (e.g., magnesium stearate, talc, or silica); disintegrants (e.g., potato starch or sodium starch glycolate); or wetting agents (e.g., sodium lauryl sulphate). The tablets may be coated by methods well-known in the art. Liquid preparations for oral administration may take the form of, but are not limited to, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives, or hydrogenated edible fats); emulsifying agents (e.g., lecithin or acacia); non-aqueous vehicles (e.g., almond oil, oily esters, ethyl alcohol, or fractionated vegetable oils); and preservatives (e.g., methyl or propyl- p-hydroxybenzoates or sorbic acid). The preparations may also contain buffer salts, flavoring, coloring, and sweetening agents as appropriate. Preparations for oral administration may be suitably formulated for slow release, controlled release, or sustained release of a prophylactic or therapeutic agent(s).

The present invention is further illustrated by the following figures and examples without being limited thereto.

Figures:

Fig. 1: Logistic dose response curves the 6 most potent plant extracts, determined by Polar Screen PPAR Competitive Assay.

Fig. 2: Logistic dose response curves of the 6 most potent unconcentrated plant extracts, determined by Polar Screen PPAR Competitive Assay.

Fig. 3: Logistic dose response curves of further plant extracts, determined by Polar Screen PPAR Competitive Assay.

Fig. 4: Logistic dose response curves of isolated compounds of different plant extracts, determined by Polar Screen PPAR Competitive Assay.

Fig. 5: eNOS activity of plant extracts

Fig. 6: results of eNOS assay

Fig. 7: results of nitrite assay

Example 1: PPAR-gamma assay and PPAR-gamma binding activity

1.1 Extraction and preparation of the plants/plant powders

100 mg dry powder of plants, herbs or spices has been suspended in 1 ml DMSO for 24 h at room temperature. The suspension was stirred on a magnetic stirrer. After 24h the extract was clarified by centrifugation for 1 h at 13.000 rpm. The clear supernant was further diluted by DMSO and then binding to PPAR□ was tested by Peroxisome Proliferator-Activated Receptor-gamma Competitor Assay Kit, Green (Invitrogen, Carlsbad, CA).

1.2 Polar Screen PPAR Competitive Assay

PPAR-gamma Competitive assay was performed according to manufacturers instructions as described in the PolarScreen™ PPAR Competitor Assay, Green Protocol (839-0412498 060904). PPAR-gamma -LBD and the fluorescent PPAR-gamma ligand (Fluormone™ PPAR Green) form a PPAR-gamma -LBD/Fluormone™ PPAR-gamma Green complex which has a high polarization value. If a competitor of PPAR-gamma is added the fluorescent ligand is displaced. This causes that the Fluoromone is free in solution and tumbles rapidly during its fluorescence lifetime which results in a low polarisation value. Substances which do not compete will not displace the fluorescent ligand from the complex so that the high polarisation value remains.

1 μl of test compounds which was dissolved and diluted in DMSO, was transferred into a microwell plate. Complete screening buffer was prepared by addition of 0.5 μl DTT (1M) to 1 ml PPAR Green Screening Buffer. 19 μl of this buffer was added to each well of microwell plate. The PPAR-LBD/Fluoromone Green complex solution was prepared by addition of 16 μl Fluoromone PPAR Green solution (500nM) per 1.6 ml and 2 μl PPAR-gamma-Ligand binding domain (1.56 mg/ml) per 1.6 ml to complete screening buffer. 20 μl of this solution was added to each well. The plate is covered to protect the reagents from light and incubated for two hours at room temperature. Fluorescence polarization is measured at excitation wavelength 485nm and emission 535 nm with Tecan Genios Pro plate reader (Tecan, Austria).

1.3 Curve fitting

Data of the ligand binding assay were calculated in the following way: Polarization values were plotted against the concentration of test compound. The curve was fitted using a logistic dose-response model from Table Curve 2D software (Jandel Scientifiec). The used function is:

$$y = a + \frac{b}{1 + (c/x)^d}$$

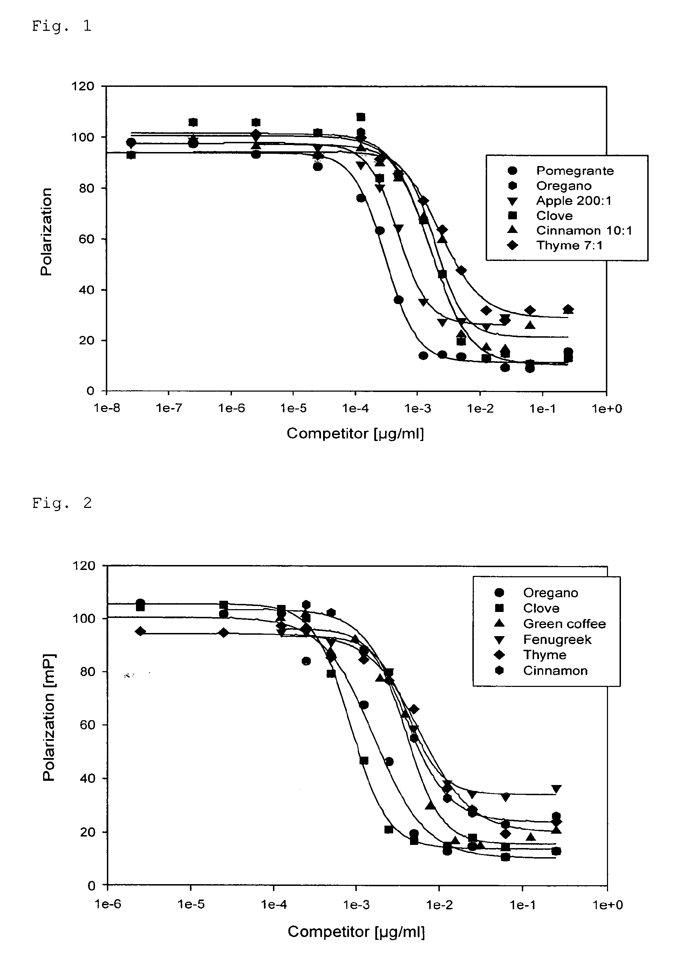

The parameter a equals the baseline, b is the difference between the plateau of the curve and the baseline and c is the transition centre of the curve which is the concentration that causes 50% of efficiency (ligand potency). *d* gives the transition zone and is a measure for positive or negative cooperativity. The concentration of the test compound that results in a half-maximal shift in polarization value is the EC50 value of the test compound. This value serves as measure of relative binding affinity to PPAR-gamma. Fitted curves are depicted in figs. 1 to 4.

Table 1: List of tested substances:

| Substance | $EC_{50}$ |
|---|---|
| CW9662 (2-Chloro-5-nitrobenanilide) | 10,7 nM |
| Rosiglitazone | 120 nM |
| Pioglitazone | 253 nM |
| Troglitazone | 1,8 $\mu$M |
| Quercetin | 2,6 $\mu$M |
| 2-Hydroxychalcone | 2,9 $\mu$M |
| Luteolin | 4,0 $\mu$M |
| Rosmarinic acid | 7,8 $\mu$M |
| Coumestrol | 8,5 $\mu$M |
| Cinnamaldehyde | 10,4 $\mu$M |
| Diosmetin | 13,7 $\mu$M |
| Biochanin A | 20 $\mu$M |
| 2'-Hydroxychalcone | 20 $\mu$M |
| Phloretin | 20 $\mu$M |
| Genistein | 22 $\mu$M |
| Ciglitazone | 23 $\mu$M |
| Isoquercetrin | 52 $\mu$M |
| Myricetin | 53 $\mu$M |
| Equol | 60 $\mu$M |
| Eriodictyol | 66 $\mu$M |
| ODMA (0-Desmethyl-angolenain) | 67 $\mu$M |
| Resveratrol | 81 $\mu$M |
| Catechin | 85 $\mu$M |
| Apigenin | 104 $\mu$M |
| Gallic acid | 128 $\mu$M |
| Galangin | 143 $\mu$M |
| Naringin | 257 $\mu$M |
| Eugenol | 420 $\mu$M |
| Kaempferol | 467 $\mu$M |
| Daidzein | 470 $\mu$M |
| Thymol | 695 $\mu$M |
| Carvacrol | 723 $\mu$M |
| Safrol | 2,25 mM |
| Ethylcinnamate | 2,70 mM |

(continued)

| Substance | EC$_{50}$ |
|---|---|
| Limonene | 9,59 mM |
| Phloridzin | >500 μM |
| Epicatechin | >500 μM |
| Enterodiol | >500 μM |
| Chrysin | >500 μM |
| 3-Hydroxyflavon | >500 μM |
| Enterolacton | >500 μM |
| Hesperidin | >500 μM |
| Capsaicin | >500 μM |
| Indol-3-Carbinol | >500 μM |
| p-Coumaric acid | >500 μM |
| Hyperoside | does not bind |
| Curcumin | does not bind |
| Sesamin | does not bind |
| Diosmin | does not bind* |
| Tamoxifen | does not bind* |
| Caffeic acid | does not bind* |
| Coumestan | does not bind* |
| Procyanidin B2 | does not bind* |
| Prunetin | does not bind* |
| Daidzin | does not bind* |
| Genistin | does not bind* |
| Ononin | does not bind* |
| Sissotrin | does not bind* |
| Puerarin | does not bind* |
| Formononetin | does not bind* |
| ICI | does not bind* |
| o-Coumaric acid | does not bind* |
| Chlorogenic acid | does not bind* |
| Estron | does not bind* |
| Protocatechuic acid | does not bind* |
| Linalool | does not bind* |
| β-Caryophyllene | does not bind* |
| Ocimene | does not bind* |
| p-Cymene | does not bind* |
| 2-Heptanon | does not bind* |
| Alliin | does not bind* |
| Diallylsulfide | does not bind* |

(continued)

| Substance | $EC_{50}$ |
|---|---|
| 2'-OH-Chalcone | does not bind |
| Ferrulic acid | does not bind |
| α-Humulene | does not bind* |
| *tested to a concentration of 0.1 M | |

Table 2: Plant extracts

| Plant, constrated extract fold | supplier | $EC_{50}$ |
|---|---|---|
| pomegranate (fruit) 40% ellagic acid | Styrka Botanics | 307 ng/ml |
| apple 200:1 5% Quercetin, 30 % Phloridz-in | Pfannnenschmidt | 467 ng/ml |
| clove | Kotany | 0,85 μg/ml |
| oregano vulgare ssp. vulgare | Kotany | 1, 6 μg/ml |
| oregano vulgare ssp. vulgare | Spar | 1,8 μg/ml |
| cinnamon 10:1 | Eurochem | 1,94 μg/ml |
| thyme 7:1 | EuroChem | 2,26 μg/ml |
| Oregano vulgare | McCormick | 3,21 μg/ml |
| cinnamon | Kotany | 3,75 μg/ml |
| green coffee | Exxentia | 4,11 μg/ml |
| Trigonella | Calendula | 4,49 μg/ml |
| blueberry, 35% phenole, 10% anthocyanine | Pharmalink | 4,55 μg/ml |
| thyme | Kotany | 6,10 μg/ml |
| nutmeg | Kotany | 6,28 μg/ml |
| rooibos tea | | 6,35 μg/ml |
| red clover blossom extract 20% | | 7,46 μg/ml |
| bay leaves | Kotany | 9,81 μg/ml |
| oregano blossom | | 10,93 μg/ml |
| red clover extract 40% | | 12,88 μg/ml |
| Origanum vulgare vulgare (Oregani cretici) | Galke | 15,13 μg/ml |
| tulsi 5:1, tannins | Pfannenschmidt | 16,10 μg/ml |
| tulsi 5:1, 1% ursol acid | Pfannenschmidt | 16,37 μg/ml |
| blue berry 5:1 | Pharmalink | 17,35 μg/ml |
| crimson clover blossoms | Südburgenland | 18,44 μg/ml |
| holy basil | Galke | 25,34 μg/ml |
| oregano 20:1 | Exxentia | 28,00 μg/ml |
| caraway | Kotany | 28,23 μg/ml |
| oregano vulgare | Fuchs | 28,55 μg/ml |
| stevia tea | | 28,78 μg/ml |
| cacao | Bensdorf | 32,36 μg/ml |
| oregano 15:1 | Exxentia | 32,75 μg/ml |

(continued)

| Plant, constrated extract fold | supplier | EC$_{50}$ |
|---|---|---|
| Mexican oregano | | 39,32 μg/ml |
| marjoram | Kotany | 39,80 μg/ml |
| tulsi | Galke | 40,18 μg/ml |
| oregano vulgare (greenware) | Exxentia | 43,21 μg/ml |
| Matai fungi | | 53,75 μg/ml |
| Helichrysum italicum (curry plant) | garden (Mühlviertel) | 54,92 μg/ml |
| rosemary | Kotany | 54,58 μg/ml |
| tarragon | garden (Mühlviertel) | 56.86 |
| rosemary 2,4 % rosemary acid | Exxentia | 56,95 μg/ml |
| ginseng | Exxentia | 57,71 μg/ml |
| kale extract | NCI | 65,65 μg/ml |
| oregano vulgare | Hamburger Gewürzmühle | 65,73 μg/ml |
| Turkish oregano türkisch | Paul Bruns | 70,23 μg/ml |
| black pepper | Kotany | 71,02 μg/ml |
| menoflavone | Melbrosin | 79,53 μg/ml |
| onion, red | NCI | 84,00 μg/ml |
| oregano 15:1 | Naturex | 95,53 μg/ml |
| santolina chamaecyarissus | garden (Mühlviertel) | 100,04 μg/ml |
| oregano vulgare | garden (Mühlviertel) | 100,11 μg/ml |
| thyme | Fuchs | 109,29 μg/ml |
| borage blossom | garden (Mühlviertel) | 114,5 μg/ml |
| fucus | | 116,6 μg/ml |
| alfalfa | Pfannenschmidt | 137 μg/ml |
| dill | Kotany | 197 μg/ml |
| nasturtium | garden (Mühlviertel) | 223,7 μg/ml |
| Jiaogulan tea | | 241,01 μg/ml |
| white cabbage extract | NCI | 289 μg/ml |
| Mulberry tea | | 290,26 μg/ml |
| marigold blossom | garden (Mühlviertel) | 323,91 μg/ml |
| oregano 4:1 | Pfannenschmidt | 388 μg/ml |
| alfalfa sprouts | health food shop | 437 μg/ml |
| lucerne (Grünmehl-Extrakt) | pet shop | 486 μg/ml |
| opuntia | Pfannenschmidt | -960 μg/ml |
| sauerkraut juice | Ja natürlich | >500 μg/ml |
| oregano 5:1 | Exxentia | >500 μg/ml |
| wood garlic | | >500 μg/ml |
| currant | Styrka Botanics | >500 μg/ml |
| lovage | garden (Mühlviertel) | > 500 μg/ml |

(continued)

| Plant, constrated extract fold | supplier | EC$_{50}$ |
|---|---|---|
| trigonella | Styrka Botanics | >500 μg/ml |
| trigonella | Fenulife | >500 μg/ml |
| ginseng | anhui | >500 μg/ml |
| phaseolus vulgaris | Linnea | >500 μg/ml |
| chive | NCI | >500 μg/ml |
| parsley | NCI | >500 μg/ml |
| leaves of santolina chamaec-yarissus | Südburgenland | >500 μg/ml |
| lavender blossom | | >500 μg/ml |
| Kudzu | | does not bind |
| cress | | does not bind |
| kvass | | does not bind |
| celeriac must | | does not bind |

Table 3: Active substances in Oregano

| Substance | EC50 |
|---|---|
| Quercetin | 2,6 μM |
| Luteolin | 4,0 μM |
| Rosmarinic acid | 7,8 μM |
| Diosmetin | 13 μM |
| Biochanin A | 20 μM |
| Isoquercetrin | 53 μM |
| Eriodictyol | 66 μM |
| Narigenin | 81 μM |
| Apigenin | 104 μM |
| Vitexin | 156 μM |
| Naringin | 257 μM |
| Taxifolin | 275 μM |
| Salvianolic acid B | 292 μM |
| Chrysoeriol | 323 μM |
| Kaempferol | 467 μM |
| Thymol | 695 μM |
| Carvacrol | 723 μM |
| Limonene | 9,59 mM |
| PeonidinCl | >500 μM |
| Ursolsäure | >500 μM |
| Apigenin-7Glycoside | >500 μM |
| p-Coumaric acid | >500 μM |
| Vanillic acid | >500 μM |

Table 4: PPAR-γ binding assays of different oregano batches (supplier: Kotany)

| extract | EC$_{50}$ |
|---------|-----------|
| Oregano 1 | 1, 6 µg/ml |
| Oregano 2 | 1,8 µg/ml |
| Oregano 3 | 3,4 µg/ml |
| Oregano 4 | 34 µg/ml |
| Oregano 5 | 35 µg/ml |
| Oregano 6 | 41 µg/ml |

Example 2: PPAR-gamma activation of wines

The wines described in table 7 have been tested as described in example 1.

Table 5 : EC$_{50}$ values of PPAR-gamma ligand binding assays of selected red wines. It was assumed that each wine contains 20g/l dry substance.

| | EC$_{50}$: without concentration, filtered | EC$_5$0 : Normalised for 20 g/l dry substance |
|---|---|---|
| Chianti classico, Italy | 707 nl/ml | 14 µg/ml |
| Valpolicella, Italy | 440 nl/ml | 8,8 µg/ml |
| Malbec, Argentina | 436 nl/ml | 8,7 µg/ml |
| Bordeaux, France | 22 µl/ml | 435 µg/ml |
| Zinfandel, California | 44 µ/ml | 888 µg/ml |
| Zweigelt Reserve, Austria | 87 µl/ml | 1,7 mg /ml |
| Zweigelt classic, Austria | 120 µl/ml | 2,4 mg/ml |
| Cabernet sauvignon, Chile | 34 µl/ml | 682 µg/ml |

Example 3: eNOS activation

Cell cultures and treatments

Human umbilical vein endothelial cells (HUVECs) were harvested enzymatically with type I A collagenase (1 mg/ml) as previously described (Simoncini et al., 1999) and maintained in phenol red-free DMEM, containing HEPES (25 mM), heparin (50 U/ml), endothelial cell growth factor (50 ng/ml), L-glutamine (2 mM), antibiotics, and 10% fetal bovine serum (FBS). Before each experiment, HUVECs were kept for at least 48 h in DMEM containing 10% steroid-deprived FBS. All experiments were performed on confluent monolayers of endothelial cells. Before every experiment investigating rapid, nontranscriptional effects (up to 30-min treatments), HUVECs were serum starved in DMEM containing no FBS for 8 h before treatment to avoid the confounding effects of serum. Inhibitor were added 30 min before the treatments.

eNOS activity assay

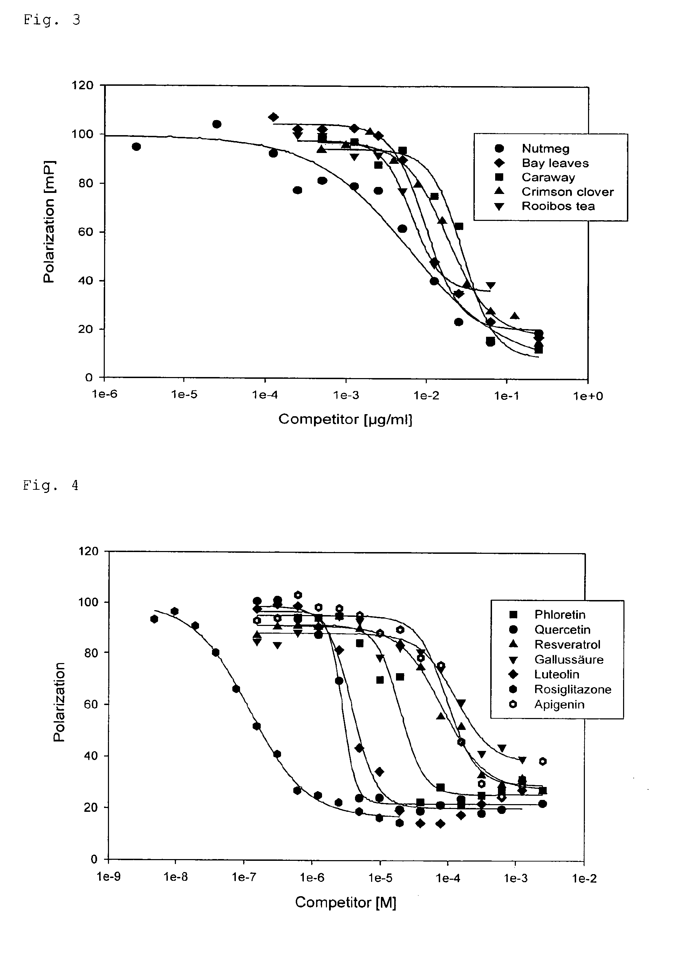

eNOS activity was determined as conversion of [³H]arginine to [³H]citrullinein endothelial cell lysates. Converted citrulline was separated by unconverted arginine using the acidic ionexchange resin Dowex 50 W, 200-400, as described (Simoncini et al., 2000). Extracts incubated with the eNOS inhibitor, N-nitro-L-arginine methyl ester (1 mM), served as blank (Fig. 2).

Nitrite assay

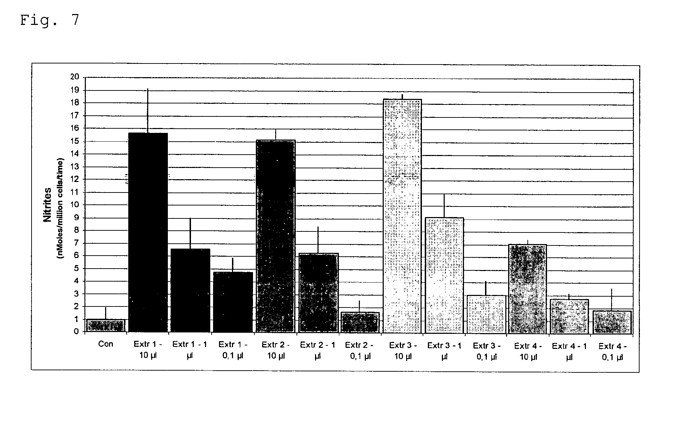

Nitric oxide production was determined by a modified nitrite assay using 2,3-diaminonaphtalene as described (Simoncini and Genazzani, 2000). Fluorescence of 1-(*H*)-naphtotriazole was measured with excitation and emission wavelengths of 365 and 450 nm. Standard curves were constructed with sodium nitrite (Fig. 7). Nonspecific fluorescence was determined in the presence of NG-monomethyl-L-arginine (3 mM).

Sample 1: Diazimt
Sample 2: Thymian7:1
Sample 3: Oregano (Kotany)
Sample 4: cinnamon (Kotany)

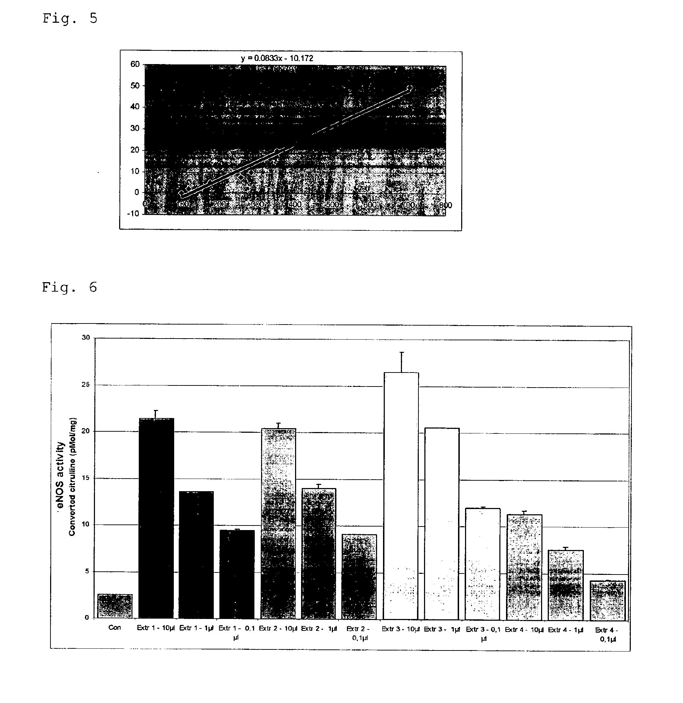

Table 6: Values of the nitrite standard curve as illustrated in Fig. 5.

| Nitrites | | | |
|---|---|---|---|
| | extracts dose dependency 48 hours | | |
| Concentration in $\mu$M | Nitrite (standard curve) | | mean |
| 0 | 100 | 99 | 99.5 |
| 2 | 123 | 114 | 118.5 |
| 5 | 177 | 172 | 174.5 |
| 7.5 | 250 | 263 | 256.5 |
| 10 | 277 | 290 | 283.5 |
| 20 | 338 | 367 | 352.5 |
| 50 | 688 | 720 | 704 |

Table 7: modifying effects of plant extracts on the activity of eNOS - illustrated in Fig. 6:

| Sample - concentration | eNOS activity pMol/mg |
|---|---|
| blank | 2.561628 |
| Extr 1 - 10 $\mu$l | 21.4162 |
| Extr 1 - 1 $\mu$l | 13.56301 |
| Extr 1 - 0,1 $\mu$l | 9.481734 |
| Extr 2 - 10 $\mu$l | 20.3668 |
| Extr 2 - 1 $\mu$l | 14.01594 |
| Extr 2 - 0,1 $\mu$l | 9.056034 |
| Extr 3 - 10 $\mu$l | 26.45035 |
| Extr 3 - 1 $\mu$l | 20.50787 |
| Extr 3 - 0,1 $\mu$l | 11.94931 |
| Extr 4 - 10 $\mu$l | 11.27611 |
| Extr 4 - 1 $\mu$l | 7.501733 |
| Extr 4 - 0,1 $\mu$l | 4.242154 |

eNOS results

The effect of oregano extract, thyme extract and two different cinnamon extracts on NO synthesis and endothelial nitric oxide synthase (eNOS) activity in human endothelial cell was tested (Fig. 6). Different amounts of plant extracts (100mg/ml DMSO) were administered to serum-starved, steroid-deprived human umbilical vein endothelial cells (HUVECs) and incubated for 48 hours. Exposure to oregano extract resulted in a concentration-dependent increase in NO release in the cell culture medium which was associated with a parallel activation of eNOS.

In comparison to thyme and cinnamon extract oregano had a higher influence on eNOS activation. 1 µl of oregano extract (100 mg/ml) had higher activation potential as 10 µl thyme or 10 µl cinnamon extract (100 mg/ml).

**Literature**

Grundy, Nat. Rev. Drug Disc. 5 (2006): 295-309
Gurnell et al., Journal of Clinical Endocrinology and Metabolism (88) (2003): 2412-2421
Koukoulitsa et al.,Bioorganic and Medicinal Chemistry (14) (2006): 1653-1659
Lemhadri et al., Journal of Ethnopharmacology (92) (2004): 251-256
Guo et al., Pharmacology and Therapeutics 111(1) (2006): 145-73
McCue et al., Asia Pacific Journal of Clinical Nutrition (13) (2004): 401-408
McCue et al., Asia Pacific Journal of Clinical Nutrition (13) (2004): 101-106
Yaniv et al., Journal of Ethnopharmacology (19) (1987): 145-151
Simoncini et al. Journal of Clinical Endocrinology and Metabolism 84 (1999): 2802-2806.
Simoncini and Genazzani. Journal of Clinical Endocrinology and Metabolism 85 (2000): 2966-2969.
Simoncini et al. Nature 407 (2000): 538-541.

**Claims**

1. Use of a plant extract or plant juice for the production of a pharmaceutical or nutritional preparation for the treatment or prevention of metabolic syndrome, wherein the plant extract or juice is from thyme, oregano, glove, nutmeg, red clover, bay leaves, red onion or grapes.

2. Use according to claim 1, **characterized in that** the plant extract or juice is from oregano.

3. Use according to claim 1 or 2, **characterized in that** the plant extract or juice has an $EC_{50}$-value of the PPAR-gamma binding of less than 50 µg/ml, preferably less than 35 µg/ml, more preferred less than 20 µg/ml, even more preferred less than 10 µg/ml, especially preferred less than 5 µg/ml, most preferred less than 1 µg/ml based on the dry-weight of the plant.

4. Use of isolated compounds selected from Quercetin, 2-Hydroxychalcone, Luteolin, Cinnamaldehyde, Diosmetin, 2'-Hydroxychalcone, Phloretin, Isoquercetrin, Myricetin, Equol, Eriodictyol, ODMA (O-Desmethyl-angolenain), Resveratrol, Catechin, Apigenin, Vitexin, Gallic acid, Galangin, Naringin, Eugenol, Eriodictyol, Apigenin, Taxifolin, Salvianolic acid B, Chrysoeriol, Kaempferol, Thymol, Carvacrol, Safrol, Ethylcinnamate, Limonene or mixtures thereof, preferably selected from Quercetin, Luteolin, Diosmetin, Isoquercetrin, Eridictoyl, Naringenin, Eriodictyol, Apigenin, Vitexin, Taxifolin, Salvianolic acid B, Chrysoeriol, Kaempferol, Thymol, Carvacrol or mixtures thereof, for the production of a pharmaceutical or nutritional preparation for the treatment or prevention of metabolic syndrome.

5. Use according to claim 4, **characterized in that** the compounds of the preparation have an $EC_{50}$-value of the PPAR-gamma binding of less than 900 µM, preferably less than 500 µM, more preferred less than 150 µM, even more preferred less than 70 µg/ml, especially preferred less than 55 µM, most preferred less than 15 µM.

6. Use according to any one of claims 1 to 5, **characterized in that** the metabolic syndrome is associated with a lack of activation of PPAR-gamma.

7. Use according to any one of claims 1 to 6, **characterized in that** the metabolic syndrome is associated with diabetes, obesity, dyslipidaemia, hypolipidaemia, insuline resistence or arteriosclerosis.

8. Use according to claim 1 to 7, **characterized in that** metabolic syndrome is associated with the clinical symptoms of diabetes type II.

9. Use according to any one of claims 1 to 8, **characterized in that** compounds of the preparation have an eNOS activity of 3 pMol/mg to 40 pMol/mg, preferably of 4 to 35 pMol/mg, even more preferred of 5 pMol/mg to 30 pMol/mg, most preferred of 6 pMol/mg to 25 pMol/mg.

**10.** Use according to any one of claims 1 to 9, **characterized in that** the extract, juice or compounds are used for the production of a pharmaceutical or nutritional preparation for the treatment of metabolic syndrome and at least one of selected from cardiovascular disease and stenosis.

**11.** Use according to any one of claims 1 to 10, **characterized in that** the preparation is an oral preparation, preferably in form of a juice or tablet.

Fig. 1

Fig. 2

Fig. 3

Fig. 4

Fig. 5

Fig. 6

Fig. 7

**European Patent Office**

**EUROPEAN SEARCH REPORT**

Application Number

EP 06 45 0165

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KWON YOUNG-IN I ET AL: "Evaluation of clonal herbs of Lamiaceae species for management of diabetes and hypertension." ASIA PACIFIC JOURNAL OF CLINICAL NUTRITION 2006, vol. 15, no. 1, 2006, pages 107-118, XP009080781 ISSN: 0964-7058 * abstract * * page 116, column 2, paragraph 1 * ----- | 1-3,6-11 | INV. A61K36/185 A61K36/53 A61P3/10 |
| D,X | MCCUE PATRICK ET AL: "Inhibitory effect of clonal oregano extracts against porcine pancreatic amylase in vitro." ASIA PACIFIC JOURNAL OF CLINICAL NUTRITION 2004, vol. 13, no. 4, 2004, pages 401-408, XP000908077 ISSN: 0964-7058 * abstract * * page 407, column 1, paragraph 2 - column 2, paragraph 1 * ----- | 1-3,6-11 | |
| X,D | LEMHADRI A ET AL: "Anti-hyperglycaemic activity of the aqueous extract of Origanum vulgare growing wild in Tafilalet region" JOURNAL OF ETHNOPHARMACOLOGY, vol. 92, no. 2-3, June 2004 (2004-06), pages 251-256, XP002425921 ISSN: 0378-8741 * abstract * ----- -/-- | 1-3,6-11 | TECHNICAL FIELDS SEARCHED (IPC) A61K A61P |

The present search report has been drawn up for all claims

| Place of search | Date of completion of the search | Examiner |
|---|---|---|
| Munich | 21 March 2007 | Vandenbogaerde, Ann |

EPO FORM 1503 03.82 (P04C01)

**European Patent Office**

## EUROPEAN SEARCH REPORT

Application Number

EP 06 45 0165

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GROVER J K ET AL: "MEDICINAL PLANTS OF INDIA WITH ANTI-DIABETIC POTENTIAL" JOURNAL OF ETHNOPHARMACOLOGY, ELSEVIER SCIENTIFIC PUBLISHERS LTD, IE, vol. 81, no. 1, June 2002 (2002-06), pages 81-100, XP009058796 ISSN: 0378-8741 * abstract * * page 82, column 2, last paragraph - page 83, column 1, paragraph 3 * | 1 | |
| X | AJAY MACHHA ET AL: "Effect of quercetin on altered vascular reactivity in aortas isolated from streptozotocin-induced diabetic rats." DIABETES RESEARCH AND CLINICAL PRACTICE JUL 2006, vol. 73, no. 1, July 2006 (2006-07), pages 1-7, XP002425922 ISSN: 0168-8227 * abstract * | 4,5 | |

TECHNICAL FIELDS SEARCHED (IPC)

The present search report has been drawn up for all claims

| Place of search | Date of completion of the search | Examiner |
|---|---|---|
| Munich | 21 March 2007 | Vandenbogaerde, Ann |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005027661 A
- CA 2526589
- WO 2005053724 A
- US 20060030597 A1
- CA 2526589 A1
- EP 1350516 B1
- JP 2005097216 A

**Non-patent literature cited in the description**

- **GRUNDY.** *Nat. Rev. Drug Disc.,* 2006, vol. 5, 295-309
- **GURNELL et al.** *Journal of Clinical Endocrinology and Metabolism,* 2003, (88), 2412-2421
- **KOUKOULITSA et al.** *Bioorganic and Medicinal Chemistry,* 2006, (14), 1653-1659
- **LEMHADRI et al.** *Journal of Ethnopharmacology,* 2004, (92), 251-256
- **GUO et al.** *Pharmacology and Therapeutics,* 2006, vol. 111 (1), 145-73
- **MCCUE et al.** *Asia Pacific Journal of Clinical Nutrition,* 2004, (13), 401-408
- **MCCUE et al.** *Asia Pacific Journal of Clinical Nutrition,* 2004, (13), 101-106
- **YANIV et al.** *Journal of Ethnopharmacology,* 1987, (19), 145-151
- **SIMONCINI et al.** *Journal of Clinical Endocrinology and Metabolism,* 1999, vol. 84, 2802-2806
- **SIMONCINI ; GENAZZANI.** *Journal of Clinical Endocrinology and Metabolism,* 2000, vol. 85, 2966-2969
- **SIMONCINI et al.** *Nature,* 2000, vol. 407, 538-541